## Europäisches Patentamt

## European Patent Office

## Office européen des brevets

(11) Publication number: **0 182 502**
**B1**

(12) **EUROPEAN PATENT SPECIFICATION**

(45) Date of publication of patent specification: **06.02.91**

(51) Int. Cl.⁵: **A 61 M 5/14**

(21) Application number: **85307566.1**

(22) Date of filing: **18.10.85**

(54) Drug delivery system.

(30) Priority: **19.10.84 US 663050**
**19.10.84 US 663051**
**27.08.85 US 769879**

(43) Date of publication of application:
**28.05.86 Bulletin 86/22**

(45) Publication of the grant of the patent:
**06.02.91 Bulletin 91/06**

(84) Designated Contracting States:
**AT BE CH DE FR GB IT LI LU NL SE**

(56) References cited:
**EP-A-0 069 350**
**EP-A-0 110 687**
**DE-A-3 138 267**
**DE-A-3 343 708**
**US-A-4 410 322**

(73) Proprietor: **Pharmacia Deltec, Inc.**
**1265 Grey Fox Road**
**St. Paul, Minnesota 55112 (US)**

(72) Inventor: **Berg, Harvey F.**
**8147 Fawn Lake Drive**
**Stacy Minnesota 55079 (US)**
Inventor: **Leslie, James E.**
**7647 Knollwood Drive N.E.**
**Mounds View Minnesota 55432 (US)**
Inventor: **Peterson, Lyle**
**10437 Valley Forge Lane**
**Maple Grove Minnesota 55369 (US)**
Inventor: **Doan, Phong**
**155 Wendy Court**
**Shoreview Minnesota 55112 (US)**

(74) Representative: **Mayes, Stuart David et al**
**BOULT, WADE & TENNANT 27 Furnival Street**
**London, EC4A 1PQ (GB)**

Courier Press, Leamington Spa, England.

## Description

The present invention relates to apparatus for delivering a drug to a patient, comprising a reservoir module for holding a container for storing the drug and means for providing communication of the drug between the container and the patient, a control module for controlling delivery of the drug, which control module is provided separably from said reservoir module and which comprises means for forcing the drug from the container to the patient at desired rates, and means for removably securing the two modules together. An example of such apparatus is seen in EP—A—0110687.

The present invention aims to improve upon such known apparatus and the invention is characterised in that the reservoir module further comprises a pressure plate for supporting the communication providing means, and in that the drug forcing means of the control module operates by interaction with the communication providing means located on the pressure plate when the two modules are connected together.

In providing for separate reservoir and control modules in this way, the invention allows several important advantages over the prior art apparatus. In particular, there is the possibility to design the apparatus with parts which operate in simple and efficient manner and where the risks of ingress of foreign bodies or unauthorised tampering with the internal workings are eliminated.

Specific embodiments of the invention will now be described by way of example with reference to the accompanying drawings where:

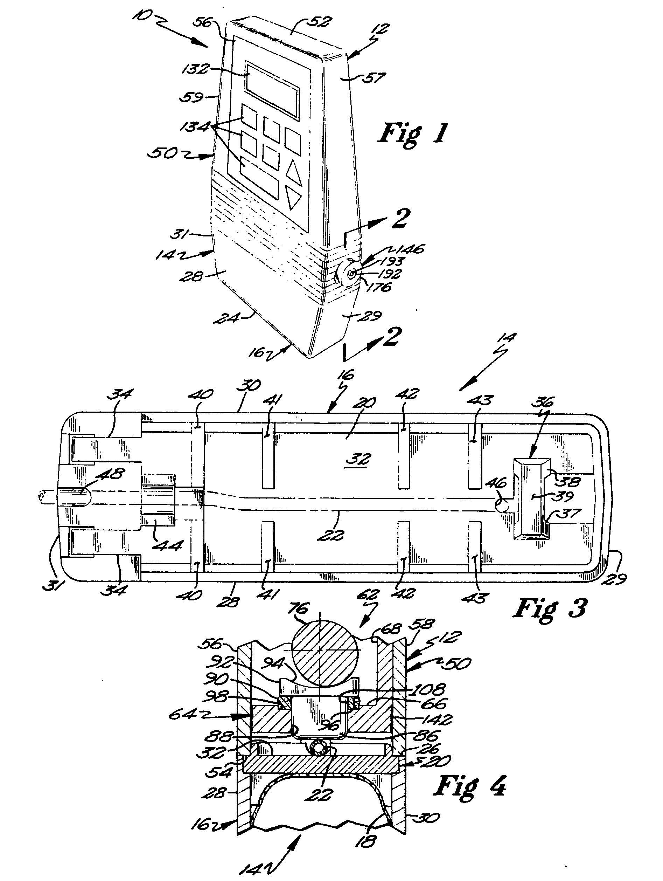

Figure 1 shows a perspective view of drug delivery apparatus according to the preferred embodiment of the present invention.

Figure 2 shows a partial, cross sectional view of the apparatus of Figure 1 according to section line 2-2 of Fiugre 1, with the reservoir module shown in its separated position in phantom.

Figure 3 shows a top view of the apparatus of Figure 1 according to view line 3-3 of Figure 2.

Figure 4 shows a partial, cross sectional view of the apparatus of Figure 1 according to section line 4-4 of Figure 2.

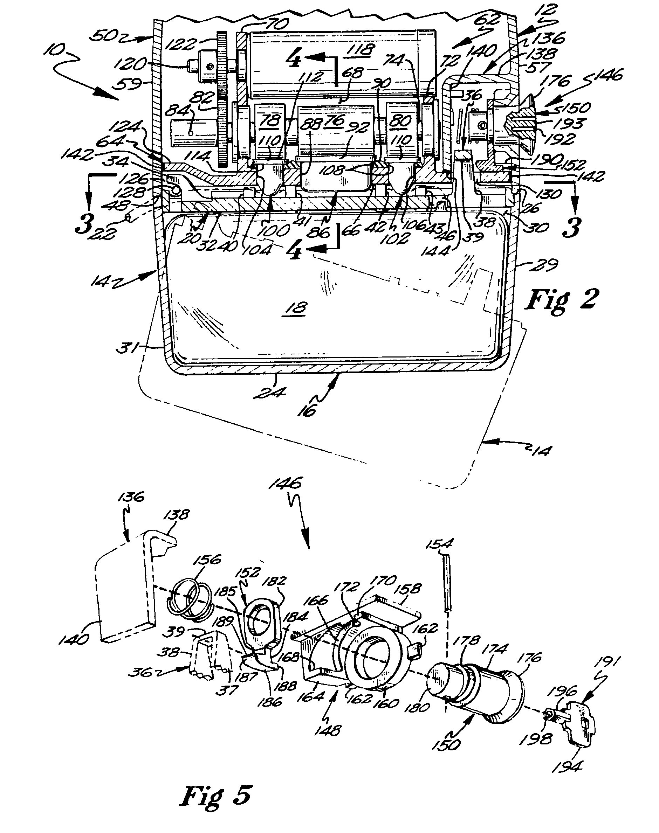

Figure 5 shows an exploded perspective view of the locking mechanism of the apparatus of Figure 1.

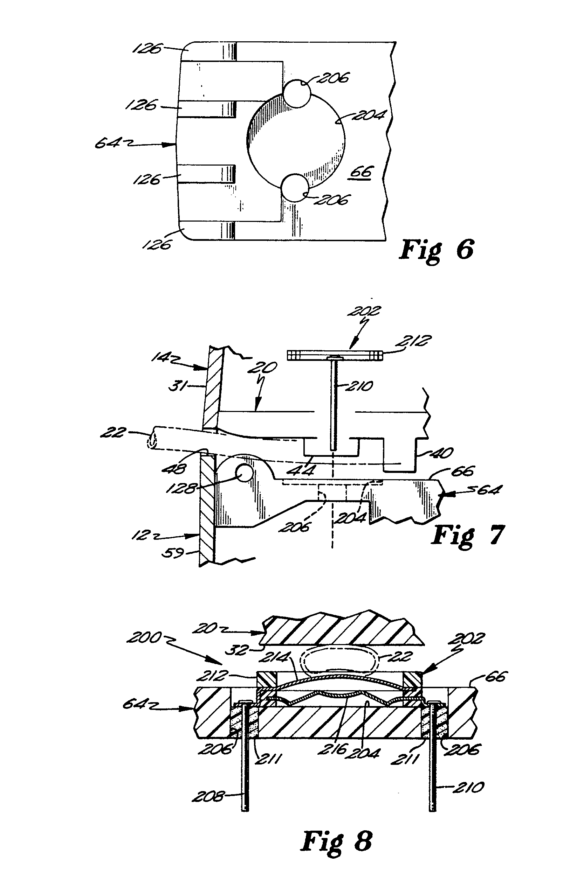

Figure 6 shows a partial, top view of the control module of an alternate embodiment of a drug delivery apparatus according to the present invention, with the switch of the occlusion detector being removed.

Figure 7 shows a partial sectional view of the apparatus of Figure 6 according to section line 7-7 of Figure 6, with the switch of occlusion detector being exploded therefrom.

Figure 8 shows a partial, sectional view of the apparatus of Figure 6.

Apparatus for providing drug delivery is shown in the drawings according to the preferred embodiment of the present invention and generally designated 10. Apparatus 10 is particularly adapted for securement to a patient such as by a belt allowing the patient to be ambulatory while having continuous infusion of a drug at the desired rate. Apparatus 10 in its most general preferred form includes a control module 12 and a reservoir module 14.

Reservoir module 14 in its most preferred form includes a reservoir casing 16, a medicament container 18 shown in its most preferred form as a heat sealed vinyl bag, and a pressure plate 20. Bag 18 further includes a member 22 for providing communication between bag 18 and an infusion catheter of the patient and is shown in its most preferred form as an elastomeric tube. In its most preferred form, casing 16 is tub shaped and includes a generally closed bottom 24, a generally open top 26, and generally closed sidewalls 28—31.

Pressure plate 20 in the preferred embodiment shown generally includes a flat portion 32 having a shape complementary to and for receipt in open top 26 of casing 16. Pressure plate 20 in its most preferred form includes hooked portions 34 which upstand from flat portion 32 at a first end thereof. In its preferred form shown, pressure plate 20 further includes an inverted U-shaped member 36 upstanding from flat portion 32 at its end opposite to hooked portions 34. Specifically, member 36 includes first and second legs 37 and 38 having their first ends connected to flat portion 32 and their second ends connected to a central portion 39 which is generally parallel to flat portion 32. Pressure plate 20 in the preferred embodiment shown further includes standoffs 40—43 upstanding from the top surface of flat portion 32 to a height for purposes set forth hereinafter. Standoffs 40—43 generally include first and second portions which extend between the sides of flat portion 32 and which are separated by a distance generally equal to the diameter of tube 22. Pressure plate 20 in the preferred embodiment shown further includes a trough portion 44 located between standoff 40 and the first end of plate 20 including hooked portions 34 having a size and shape complementary to and for receiving tube 22. In the preferred embodiment, tube 22 extends through an opening 46 formed in flat portion 32 adjacent member 36, extends between the first and second portions of standoffs 40—43, extends through trough portion 44, and through a removed portion 48 formed in the end of flat portion 32 adjacent hooked portions 34. In its most preferred form, tube 22 lies on flat portion 32 of pressure plate 20 generally midway between and parallel to the sides of flat portion 32. Tube 22 in the preferred embodiment of the present invention is secured to pressure plate 20 by a solvent bond located between tube 22 and trough portion 44 and is anchored by its other end to pressure plate 20 and reservoir module 14 by its securement to bag 18 captured between pressure plate 20 and casing 16 and its passage through opening 46 of pressure plate 20.

Control module 12 in its most preferred form includes a casing 50 shown in its most preferred from formed of first and second halves and includes a generally closed top 52, a generally open bottom 54, and generally closed sidewalls 56—59.

Control module 12 further includes a member 62 interacting with tube 22 for forcing liquid from bag 18 through tube 22 and for preventing free flow of liquid through tube 22 either to or from bag 18. Member 62 is shown in its most preferred form as a pumping mechanism. Pumping mechanism 62 includes a chassis 64 including a first flat portion 66 and a second flat portion 68 generally at right angles to each other. In the preferred form, flat portion 68 has a shorter length than portion 66 and the ends of flat portion 68 are both spaced from the respective ends of portion 66. Chassis 64 further includes braces 70 and 72 which extend beteween portion 66 and the ends of portion 68 and which are spaced from each other and the ends of portion 66.

Pumping mechanism 62 further includes in its most preferred form a camshaft 74 rotatably mounted between braces 70 and 72 and having a first cam portion 76 located between second and third cam portions 78 and 80 on shaft 74. Shaft 74 extends through brace 70 and further includes a driven gear 82 and an optical switch indicator 84 shown in its most preferred form as a flag located between the end of portion 66 and brace 70.

In the preferred embodiment, pumping mechanism 62 further includes an expulsor 86 which is slideably mounted in an aperture 88 formed in portion 66. Expulsor 86 includes a head portion 92 including an arcuate portion 94 for engaging with cam portion 76 and an abutment surface 96 which prevents expulsor 86 from passing through aperture 88 of chassis 64.

In the preferred embodiment, pumping mechanism 62 further includes first and second valves 100 and 102 which are slideably mounted in apertures 104 and 106 formed in portion 66 of chassis 64, respectively. Valves 100 and 102 each include a head portion 110 having an arcuate portion 112 for engaging with cam portions 78 and 80 and having an abutment surface 114 which prevents valves 100 and 102 from passing through apertures 104 and 106 of chassis 64.

Provisions 90 are further provided for biasing expulsor 86 and valves 100 and 102 towards cam portions 76, 78 and 80 and for sealing expulsor 86 and valves 100 and 102 with flat portion 66 of chassis 64 of control module 12. In its most preferred form, provisions 90 is a closed cell foam gasket located in a cavity 98 formed in the top surface of flat portion 66 of chassis 64 and with which head portions 92 and 100 of expulsor 86 and valves 110 and 102 abut. Gasket 90 includes apertures 108 formed therein corresponding to apertures 88, 104 and 106 of chassis 64 and having a size and shape complementary to the receipt of expulsor 86 and valves 100 and 102. Gasket 90 has sufficient resiliency to bias expulsor 86 and valves 100 and 102 and has sufficient sealing ability to seal between chassis 64 and expulsor 86 and valves 100 and 102.

In its most preferred form, pumping mechanism 62 further includes a member 118 for driving camshaft 74 shown as a motor mounted between braces 70 and 72. Motor 118 includes a drive shaft 120 which extends through brace 70 having a drive gear 122 in gearing relation with gear 82 of camshaft 74. In its most preferred embodiment, motor 118 is a D.C. motor that rotates camshaft 74 through 360 degrees and then stops, with the number of rotations of shaft 74 controlling the volume of medicament being pumped from bag 18 of reservoir module 14.

In the preferred embodiment of the present invention shown, flat portion 66 of chassis 64 includes an upraised portion 124 located adjacent its first end having depending ears 126 extending therefrom. Hinge pins 128 are further provided extending between ears 126 generally parallel to flat portion 66. Hinge pins 128 are complementary to and for hingedly receiving hooked portions 34 of plate 20 of reservoir module 14. Flat portion 66 of chassis 64 further includes an aperture 130 located adjacent its opposite end complementary to and for receiving U-shaped member 36 of plate 20 of reservoir module 14.

Control module 12 further includes a source of power for motor 118, such as a battery, and suitable electronic controls for the operation of motor 118 for controlling the pumping of the liquid from bag 18 through tube 22. Included with the electronic controls, control module 12 may include a liquid crystal display (LCD) 132 and switches or keys, generally designated 134, for purposes of programming the electronic controls to start or stop the pump, to vary the rate of pumping, for priming the pump, to limit patient operation of the pump, and like functions. For example, programming modes of apparatus 10 of the present embodiment may include: delivering drug concentrations of different rates: delivering a basal infusion at a set rate; allowing the patient the option of varying the basal rate up to the maximum rate allowed by the physician; delivering an incremental bolus dose at a set rate; allowing the patient the option of varying the incremental dose up to the maximum rate allowed by the physician; allowing the physician to set the minimum time between incremental doses; allowing the physician to limit the number of incremental doses per hour; and displaying the milligrams of medicament delivered, the number of incremental doses delivered, and the amount of volume of medicament remaining in the reservoir on the LCD 132. Thus, LCD 132 visually indicates various conditions of apparatus 10 such as the rate of pumping, the volume of liquid remaining in bag 18, and like conditions. In its most preferred form, LCD 132 and keys 134 are located in sidewall 56 of casing 50 of control module 12. The electronic controls may further include a user interaction signal such as a buzzer or other audible alarm for purposes of indicating the condition of apparatus 10 such as indicating the pressing of keys 134, a low battery, an empty reservoir, an error in programming the electronic controls, or like condition.

In other words, knowing factors such as the type and chracteristics of the pumping mechanism and the type and concentration of medication, a manufacturer of apparatus according to this embodiment would be in a position to program each apparatus by modern electronics. That is, taking into account the above factors and other factors and constraints set by modern medicine and the laws of physics, a manufacturer can program each apparatus to receive such variables as: patient dosage; medicament concentration; the medication schedule; the reservoir size; and other like considerations and can arrange the apparatus to perform such functions as: dosage control; frequency of medication; variation of medication scheduling and dosage; various warnings and conditions, such as a low battery, error in programming the electronic controls, pressing of keys 134, and an empty reservoir; residential reservoir medication amount; and other like functions for particular medications, particular patients, particular needs, etc.

Casing 50 further includes an L-shaped casing portion 136 extending from sidewall 57 and between sidewalls 56 and 58. Specifically, portion 136 includes a first leg 138 having a first end integrally connected to sidewall 57 and having a second end integrally connected to a second leg 140. The free end of leg 140 extends into open bottom 54 of casing 50.

In its most preferred form, flat portion 66 of chassis 64 has a shape and size complementary to and for receipt in open bottom 54 of casing 50. Control module 12 further includes sealing members 142 located between the ends and sides of portion 66 of chassis 64 and sidewalls 56—69 of casing 50. Further, sealing member 144 is located between the free end of leg 140 of L-shaped casing portion 136 and the top surface of flat portion 66 of chassis 64.

It can now be appreciated that with chassis 64 located in casing 50 with flat portion 66 located within open bottom 54, a substantially water tight compartment which includes motor 118, the electronic controls, the pumping mechanism 62, and other system components is defined by sidewalls 56—59, top 52, and L-shaped casing portion 136 of casing 50, flat portion 66 of chassis 64, expulsor 86, valves 100 and 102, gasket 90, and sealing members 142 and 144. Thus, the apparatus components within this water tight compartment are protected from dust, the elements, or other comtaminants which may produce damage thereto.

Apparatus 10 in the preferred embodiment includes provisions 146 for removably holding the reservoir module 14 to the control module 12 and is shown in its most preferred form as a locking mechanism. Locking mechanism 146 generally includes, in the preferred embodiment, a latch guide 148, a latch button 150, a locking bolt 152, a pin 154, and a spring 156. Latch guide 148 generally includes a flat portion 158 which is captured between the halves of casing 50. A cylindrical member 160 is further provided depending from the end of flat portion 158 for rotatably and slideably receiving latch button 150. Ears 162 are provided on cylindrical member 160 for capturing between the halves of casing 50. A longitudinally extending, arcuate portion 164 is further provided on latch guide 148 depending from flat portion 158 and having a first end secured to cylindrical member 160. The second, free end of arcuate portion 164 includes a helical, camming portion 166 having stops 168 and 170 formed in and by arcuate portion 164. Camming portion 166 further includes a detent 172 formed adjacent stop 170 at the longitudinally inward end of camming portion 166.

Latch button 150 generally includes a shank portion 174 which is rotatably and slideably received in cylindrical member 160 of latch guide 148 about its longitudinal axis and a head portion 176 which prevents shank portion 174 from passing through cylindrical member 160 in a first direction. Latch button 150 further includes an eccentric portion 178 which is eccentrically located with respect to the longitudinal axis of shank portion 174 and an extended shank portion 180.

Locking bolt 152 generally includes a cylindrical portion 182 having a shape and size complementary to and for receipt on eccentric portion 176 of latch button 150. Locking bolt 152 further generally includes a T-shaped member 184 extending from cylindrical portion 182. T-shaped member 184 generally includes a leg member 185 having its first end connected to cylindrical portion 182 and its second end connected substantially to the middle of a cross member 186. Cross member 186 includes a first inner end 187 and a second, outer end 188. Cross member 186 includes a surface 189 located on the upper surface of cross member 186 between leg member 185 and end 187 for engaging with central portion 39 of U-shaped member 36 of pressure plate 20.

Locking bolt 152 is held on eccentric portion 178 of latch button 150 in the preferred embodiment of the present invention between a shoulder formed between shank portion 174 and eccentric poriton 178 and pin 154 extending through the extended shank portion 180 of latch button 150. Pin 154 further acts as the follower for camming portion 166 and for detent 172 of arcuate portion 164 of latch guide 148. Pin 154 also prevents shank portions 174 and 180 and eccentric portion 178 from passing through cylindrical member 160 in the opposite direction of head portion 176. In its most preferred form, when latch button 150 is located at its first, extreme outward, unlocked position, pin 154 abuts with camming portion 166 adjacent stop 168 and end 188 of T-shaped member 184 of locking bolt 152 abuts with cylindrical member 160 of latch guide 148. Pin 154 further acts as an abutment for spring 158, with the spring 158 located concentrically on latch button 150 and extending between leg 140 of L-shaped casing portion 136 and pin 154. Thus, spring 158 biases latch button 150 in an outward direction.

Locking mechanism 146 further includes provisions 190 for allowing the longitudinal movement of locking bolt 152 but preventing rotatable movement of locking bolt 152 with latch button 150. Provisions 190 are shown in the preferred embodiment of the present invention as tracks formed in the top surface of flat portion 66 of chassis 64 intermediate aperture 130 and its second end and having a shape complementary to and for slideable receipt of cross member 186 of T-shaped member 184 of locking bolt 152.

Suitable provisions 191 for rotating latch button 150 may further be provided for locking mechanism 146. In a first preferred form, provisions 191 include a cavity 192 formed in head portion 176 of latch button 150 having a concentrically located, upstanding post 193. In the preferred embodiment, cavity 192 has a multisided perimeter, such as a hexagonal cross section and post 193 has a shape such as a circular cross section. Provisions 191 further includes a key 194 having a lug end 196 having a shape complementary to and for receipt within cavity 192, with the end 196 having a cavity 198 located generally concentrically therein. Cavity 198 has a size and shape complementary to and for receipt on post 193. It can then be appreciated that locking mechanism 146 utilizing key 194 restricts the personnel who can unlock reservoir module 14 from control module 12. Specifically, keys 194 would be given only to physicians, pharmacists, and other authorized persons and not be given to patients utilizing apparatus 10 or other unauthorized persons. Thus, without keys 194, the patients would be unable to rotate latch button 150 and release locking mechanism 146.

If restricted access is not required, provisions 191 may include an arcuate cavity formed in head portion 176 of latch button 150 for receiving the edge of a coin or similar object. Thus, by turning the coin, latch button 150 may be rotated. However, provisions 191 may take other forms.

The operation of locking mechanism 146 can now be set forth and appreciated. For the sake of example, it will be assumed that U-shaped member 136 of pressure plate 20 is extending through aperture 130 of flat portion 66 of chassis 64 and latch button 150 is in its outward, unlocked position. To lock locking mechanism 146, latch button 150 may be pressed from its first position inwardly within latch guide 148 against the bias of spring 156 towards its second position. When latch button 150 is moved inwardly, locking bolt 152 secured thereto by pin 154 also moves inwardly, with surface 189 of cross member 186 of locking bolt 152 being located under the central portion 39 of U-shaped member 36 of pressure plate 20. At that time, latch button 150 may be rotated to its third position utilizing provisions 191. Due to the eccentric interaction between locking bolt 152 and latch button 150, rotation of latch button 150 causes locking bolt 152 to raise up engaging surface 189 with central portion 39. It should be noted that tracks 190 of chassis 64 prevent locking bolt 152 from rotating with latch button 150. Latch button 150 may be rotated until pin 154 abuts with stop 170 and is located within detent 172 of camming portion 166. At that time, due to its eccentric interaction, locking bolt 152 exerts an upward, locking force on central portion 39 of pressure plate 20. To enhance this exertion of an upward force, surface 189 and central portion 39 may include complementary, wedge-like tapers as in the most preferred form of the present invention. Further, with pin 154 located in detent 172, latch button 150 is prevented from accidentally rotating from its third, inward, locked position.

It should then be noted that if latch button 150 is not located in its third position such that pin 154 is not located in detent 172, pin 154 under the bias of spring 156 will ride back upon camming portion 166 until stop 168. Thus, latch button 150 to which pin 154 is secured rotates and slides back to its first, unlocked position such that locking bolt 152 does not engage with U-shaped member 36. Thus, reservoir module 14 is not secured to control module 12. The reason for this subtle feature is to insure that partial engagement of locking bolt 152 with U-shaped member 36 does not occur. The full engagement of locking bolt 152 with U-shaped member 136 in cooperation with the removable hinge member including hooked portions 34 and hinge pins 128 and standoffs 40—43 insures the proper interrelation between control module 12 and reservoir module 14 for efficient and advantageous operation of pumping mechanism 62 as will be set forth hereinafter.

To unlock locking mechanism 146 releasing locking bolt 152 from U-shaped member 36 of pressure plate 20 and thus allowing removal of reservoir module 14 from control module 12, the locking operation as set forth hereinbefore may be simply reversed.

The removable securement of reservoir module 14 to control module 12 can now be set forth and appreciated. For the sake of example, it will be assumed that reservoir module 14 is removed from control module 12, that bag 18 of reservoir module 14 is full of medicament, and that it is desired to secure reservoir module 14 to control module 12. Specifically, reservoir module 14 may be positioned in a first position with respect to control module 12 such that hooked portions 34 of pressure plate 20 engage with hinge pins 128 of chassis 64 of control module 12. Thus, in the first position, the reservoir module 14 is separable from the control module 12. Reservoir and control modules 12 and 14 may then be moved towards their second position such that hooked portions 34 of pressure plate 20 hook on hinge pins 128 of chassis 64 of control module 12. At that time, control module 12 and reservoir module 14 may be placed on a flat surface such that bottom 24 of casing 16 of reservoir module 14 rests upon the flat surface. Control module 12 may then be pressed firmly in its second position such that U-shaped member 136 of pressure plate 20 of reservoir module 14 extends through aperture 130 of chassis 64 of control module 12. Locking mechanism 146 may then be locked by sliding and rotating latch button 150 in a manner set forth hereinbefore such that locking bolt 152 engages with and raises up central portion 39 of pressure plate 20. When pin 154 is located within detent 172 of camming portion 166, reservoir module 14 is firmly locked in its second position on control module 12. Specifically, reservoir module 14 is removably secured to control module 12 by a removable hinge member including hooked portions 34 and hinge pins 128 at one end and by locking mechanism 146 at its other end. Tube 22 extends between casing 16 of reservoir module 14 and casing 50 of control module 12 through removed portion 48 formed in pressure plate 20 and casing 16.

To remove reservoir module 14 from control module 12, for example to allow replacement of a reservoir module 14 having an empty bag 18 with a reservoir module 14 having a full bag 18, the reservoir module 14 securement procedure as set forth hereinbefore may be simply reversed.

It can then be appreciated that accidental and purposeful separation of control and reservoir modules 12 and 14 is substantially prevented without unlocking locking mechanism 146. Locked in its second position, breakage of hooked portions 34, hinge pins 128, locking bolt 152, and/or U-shaped member 36 must occur before separation of reservoir module 14 from control module 12 can occur without unlocking locking mechanism 146. Accidental dropping of apparatus 10 generally will not cause such breakage. Further, if purposeful breaking force is applied to apparatus 10, breakage of one or more of these components provides evidence of unauthorized separation. Thus, apparatus 10 restricts access of the drug located in bag 18 of reservoir module 14 to authorized personnel. It can then be appreciated that apparatus 10 includes provisions for the removable securement of its modular components which are of simple design and which are easy and inexpensive to manufacture and to assemble.

The operation and subtle features of pumping mechanism 62 and other subtle features of apparatus 10 can now be set forth and appreciated. In its most preferred embodiment, each cycle of the pumping mechanism 62 includes the following valve and expulsor positions. Specifically, in the at rest, stopped, or beginning of the pump cycle, valves 100 and 102 both are in their lowermost position depressing tube 22 and expulsor 86 is at its uppermost position and thus not depressing tube 22. This particular initial position has found to be particularly advantageous since valves 100 and 102 prevent flow through tube 22 in either direction. Further, the position of expulsor 86 beginning at this uppermost position reduces tube 22 deformation since tube 22 is not depressed by the relatively large area of expulsor 86 in the rest position. Further, the position of valve 100 beginning the initiation of its upward movement reduces the starting load on pumping mechanism 62 and thus reduces the size requirement 62 on motor 118 and the energy requirements for motor 118.

Upon rotation of camshaft 74, expulsor 86 compresses tube 22 placing pressure on the medicament in tube 22 between valves 100 and 102. After a slight amount of pressure is placed on tube 22 by expulsor 86, valve 100 moves to its upper position allowing flow of medicament from tube 22 past valve 100. It can then be appreciated that placement of pressure on tube 22 before valve 100 opens substantially prevents back flow of liquid from tube 22 towards valve 102. Back flow of liquid from tube 22 towards valve 102 may cause blood to back up in the catheter of the patient to which tube 22 is connected.

Upon continued rotation of shaft 74, expulsor 86 continues its downward movement. Upon further rotation, expulsor 86 reaches its lowermost position and dwells in its lowermost position while valve 100 returns to its closed, lowermost position. This dwelling of expulsor 86 in its lowermost position also prevents back flow of medicament through tube 22 past valve 100. Upon continued rotation of shaft 74, expulsor 86 starts in its upward movement. Just prior to expulsor 86 reaching its upper position, valve 102 starts in its upward movement. When expulsor 86 reaches its uppermost position, valve 102 also reaches its uppermost position. When camshaft 74 is continued in its rotation, valve 102 moves downward, while expulsor 86 dwells in its uppermost position. Upon continued rotation of camshaft 74, valve 102 returns to its closed, lowermost position, expulsor 86 dwells at its uppermost position, valve 100 dwells at its lowermost position, and indicator 84 activates an optical switch causing the driving of shaft 74 by motor 118 to be stopped at the rest pump cycle.

It can then be appreciated that with reservoir module 14 secured to control module 12, at least one of the valves 100 and 102 engages with and compresses tube 22 against flat portion 32 of plate 20 at all times. Thus, accidental infusion of fluid through tube 22 such as under the influence of gravity or by compression of bag 18 is prevented.

It may also be appreciated that pumping mechanism 62 may rely on the recovery of tube 22 for maintaining contact of expulsor 86 and valves 100 and 102 with cam portions 76, 78 and 80, respectively, during the pumping cycle. However, as in the preferred embodiment, gasket 90 or other biasing members may be utilized to assist expulsor and valve contact with cam portions 76, 78 and 80.

It can be further appreciated that the spacing between flat portion 32 of pressure plate 20 and flat portion 66 of chassis 64 has been found, thus far, to be very important in maximizing the efficiency and advantageous operation of pumping mechanism 62. Specifically, it has been found that the travel distance of expulsor 86 and valves 100 and 102 through apertures 88, 104 and 106 of flat portion 66 is a constant system characteristic. Thus, if the spacing between pressure plate 20 and chassis 64 varies, disadvantageous operation of pumping mechanism 62 occurs. Specifically, if the spacing is too great, expulsor 86 and/or valves 100 and 102 may not compress tube 22 sufficiently or may not compress tube 22 at all. If insufficient compression of tube 22 occurs, valves 100 and 102 may not prevent flow through of medicament through tube 22 in their lowermost, closed positions. Likewise, expulsor 86 may not have sufficient stroke to compress tube 22 for forcing the desired amount of the medicament out of tube 22 past valve 100, thus greatly reducing efficiency and degrading the accuracy of apparatus 10.

On the other hand, if the spacing between chassis 64 and pressure plate 20 is too small, material from which tube 22 is formed may deform. Such deformation is undesirable for several reasons. First, tube 22 may not fully recover after compression to its uncompressed condition. This is very undesirable because this degrades the accuracy of apparatus 10. Specifically, the volume of medicament pumped by apparatus 10 is directly related to the volume of the tube being compressed by expulsor 86. If plastic deformation occurs to tube 22 due to overcompression by expulsor 86 and valves 100 and 102 of pumping mechanism 62, the volume compressed by expulsor 86 may change and thus change the volume of medicament being pumped by apparatus 10. Since the delivery of medicament at prescribed rates is critical in many medical treatments, such changes in pumping volume is very disadvantageous. Additionally, such deformation may lead to the cracking and/or rupturing of tube 22. Further, if the spacing between chassis 64 and pressure plate 20 is too small, greater force is required to compress tube 22 which is detrimental in the short run by excessive use of energy and in the long run by increased wear on the pumping mechanism 62.

It can then be appreciated that in its most preferred form, plate 20 is separable from chassis 64 due to their inclusion in separate modules 12 and 14. Thus, maintenance of the desired spacing between plate 20 and chassis 64 cannot be factory set but must be insured between control module 12 and a variety of reservoir modules 14 each having different tolerances. To insure proper space maintenance while eliminating the rquirement for individual adjustment, standoffs 40—43 are provided. In their most preferred form, standoffs 42 and 42 have a height generally equal to the desired spacing between plate 20 and chassis 64 and standoffs 40 and 43 have a height slightly less than the desired spacing between plate 20 and chassis 64. It can then be appreciated that due to the placement of hinge pins 128 and hooked portions 34 and the upward force exerted on the opposite end of pressure plate 20 by the engagement of the locking mechanism 146, pressure plate 20 is drawn against chassis 64 to insure that the spacing between chassis 64 and the pressure plate 20 is not too great. Further, with chassis 64 drawn towards pressure plate 20, standoffs 41 and 42 abut with chassis 64 to insure that the spacing between chassis 64 and pressure plate 20 is not too small. Thus, standoffs 41 and 42 prevent pressure plate 20 and tube 22 mounted thereon from moving closer to chassis 64. Therefore, standoffs 41 and 42 insure the desired spacing is maintained. In their most preferred form, standoffs 40 and 43 are spaced from chassis 64 when reservoir module 14 is secured to control module 12. However, if the tolerances of any particular reservoir module 14 is greatly different than the norm, standoffs 40 and 43 act as safeguards to prevent pressure plate 20 and tube 22 mounted thereon from moving closer to chassis 64 at their respective locations when they abut therewith. Therefore, according to the present embodiment, the desired spacing of pressure plate 20 and tube 22 mounted thereon is insured to maximize system efficiency and its advantageous operation.

It should also be appreciated that standoffs 40—43 also insure that tube 22 is positioned on plate 20 for allowing engagement of expulsor 86 and valves 100 and 102 therewith. Further, tube 22 is held between and is prevented from moving between the first and second portions of standoffs 40—43 due to the abutment of standoffs 41 and 42 and the limited spacing of standoffs 40 and 43 from chassis 64. Thus, when reservoir module 14 is secured to control module 12, engagement of expulsor 86 and valves 100 and 102 with tube 22 is insured and tube 22 cannot move from between standoffs 40—43 during valve and expulsor engagement to insure complete contact of valves 100 and 102 and expulsor 86 with tube 22 at all times.

The specific pumping mechanism 62 shown in synergistic relation to the apparatus has a number of advantages over a rotary peristaltic or roller pump. Specifically, roller pumps strip the tubing causing the tubing to advance in the direction of rotation. This necessitates anchoring the tubing near the inlet to the roller pump head. Pumping mechanism 62 compresses tube 22 without pulling it so such anchoring is not rquired. Additionally, roller pumps require the tubing to be fully occluded. This complete occlusion stresses the tubing and distorts it such that the tubing does not fully recover. The expulsor 86 of pumping mechanism 62 shown need not fully occlude tube 22 and thus stresses in tube 22 may be limited to a level that does not yield unacceptable distortion. Further, threading of the tubing for engagement with the roller head is complicated in roller pumps. Positioning of tube 22 to engage with pumping mechanism 62 can be accomplished in a simple, uncomplicated manner. Likewise, less force is required to partially occlude tube 22 in apparatus 10 than required to occlude tubing in a roller pump, which reduction of force being reflected to camshaft 74 resulting in a lower drive torque.

Further, the use of active values 100 and 102 for tube 22 of the apparatus are believed advantageous over the use of passive check valves. Passive check valves are prone to allowing undesirable flow therethrough whereas valves 100 and 102 prevent flow through. It should be noted that if undesired flow occurs from the drug container to the patient, the rate of infusion is than not controlled and precise delivery of the drug is not obtained. Further, excessive amounts of drug may be delivered to the patient. Additionally, it should also be noted that undesired flow from the patient towards the drug container may cause blood to back up the catheter of the patient, which is medically undesirable. Specifically, check valves will allow flow through by their nature in one direction whereas valves 100 and 102 of the apparatus prevent flow through tube 22 in either direction. Thus, if the reservoir bag was compressed, check valves would open and allow flow through towards the patient whereas valves 100 and 102 of the apparatus will not allow such flow through. Likewise, to reduce susceptibility to leakage, check valves have a high crack force which increases the pressure necessary for the pumping action. Valves 100 and 102 are independently driven so that there is no dependence of pressure generated by expulsor 86 such that reduced pumping pressure may be utilized.

Additionally, check valves are unreliable in seating thus also allowing flow through whereas valves 100 and 102 are reliable in occluding tube 22 for preventing such flow through. Thus, active valves 100 and 102 are clearly advantageous over passive check valves.

In its most preferred form, apparatus 10 may be removably secured to the patient such as by a belt thus allowing the patient to be ambulatory while having a drug continuously administered. Thus, the patient need not be bedridden or have his freedom of movement otherwise restricted but may lead a relatively normal life. Further, apparatus 10 allows certain intravenous therapies to be given without frequent medical attention such as in a hospital environment but in an outpatient basis. Thus, health care costs may be significantly reduced.

It can also be appreciated that the fact that apparatus 10, as disclosed, includes a modular construction featuring a removable reservoir module 14 is particularly advantageous. Specifically, module 14 may be disposable such that resterilization is not required therefor, and mistakes as to mixing of different medicaments in bag 18 and like mistakes are removed. Additionally, reservoir module 14 provides increased protection against tampering of medicaments in bag 18 included within module 14. Further, tube 22 with which pumping mechanism 62 engages is a component of and is replaced with each reservoir module 14. Thus, tube 22 which is most subject to wear and deformation is replaced with each refill greatly reducing the risk of equipment failure. Additionally, the electronic controls, pumping mechanism 62, and other components included in control module 12 remain in a sealed compartment separate from reservoir module 14 and thus are not prone to accidental contact and damage when it becomes necessary to refill or replace bag 18. Further, no adjustments are required to insure proper and efficient operation of pumping mechanism 62 when it is secured to any particular reservoir module 14. Specifically, apparatus 10 and particularly standoffs 40—43, the hinge connection including hooked portions 34 and hinge pins 128, and locking mechanism 146, automatically insures the proper interrelation between control module 12 and all reservoir modules 14 utilized for efficient and advantageous operation of pumping mechanism 62. Thus, it can be appreciated that the modular construction of apparatus 10 is more user friendly than other prior drug delivery systems.

Apparatus 10, as disclosed including a modular construction featuring a removable reservoir module 14, is further advantageous in its ability to include an occlusion detector 200 of a unique, simple, and generally fool proof nature. Specifically, it can be appreciated that if the fluid is not being forced by pumping mechanism 62 through tube 22 to the patient because of an occlusion in tube 22, problems may result from the shortage of medication delivered to the patient. Such an occlusion might be caused, for example, by formation by a thrombus at the tip of an intravenous catheter, or by inadvertent failure to open a clamp applied to tube 22. Thus, it is desirable upon the occurrence of increased pressure within tube 22 resulting from an occlusion within tube 22 to interrupt the operation of pumping mechanism 62 and to allow apparatus 10 to be checked before resuming operation of pumping mechanism 62. It should be appreciated that in the event of interruption of the operation of pumping mechanism 62, at least one of the valves 100 and 102 engages with and compresses tube 22 at all times as set forth hereinbefore and thus accidental infusion of fluid and/or back flow of blood through tube 22 is prevented.

In its most preferred form, occlusion detector 200 includes a pressure switch 202 located on chassis 64 of control module 12 of apparatus 10 which abuts directly with tube 22 located on pressure plate 20 of reservoir module 14 when reservoir module 14 is secured to control module 12. In its most preferred form, a cavity 204 is provided on flat portion 66 of chassis 64 intermediate ears 126 and aperture 104. In its most preferred form, apertures 206 are provided which extend through flat portion 66 of chassis 64 for allowing passage of wires 208 and 210 of switch 202 therethrough. In its most preferred form, switch 202 is bonded to flat portion 66 of chassis 64 within cavity 204 by any suitable means such as by an epoxy bond, an adhesive or the like. Suitable sealing means 211 such as silicon rubber may be provided within apertures 206 and around wires 208 and 210 to maintain a substantially water tight compartment for protecting the controls and components within control module 12 from dust, the elements, or other contaminants which may produce damage thereto. It can then be realized that cavity 204 allows the rapid placement of switch 202 on chassis 64 during production of control module 12 and thus lends itself to rapid, easy manufacture.

It its most preferred form, switch 202 includes an annular, insulating washer 212 and first and second, diaphragm type, normally spaced contacts 214 and 216. Contacts 214 and 216 in their most preferred form are disc shaped having their outer perimeters embedded or otherwise fixed in an electrically, longitudinally spaced condition in annular washer 212. In its most preferred form, contact 216 may include a depression, as shown, for providing a circular contact area. In its most preferred form, swtich 202 may be an ITT Schadow Disc Series Switch, Model ED. Wires 208 and 210 are electrically connected to contacts 214 and 216, respectively. It can then be appreciated that when contacts 214 and 216 are in their normally spaced condition, switch 202 is unactivated, i.e. electrical current is interrupted between wires 208 and 210 by the spaced contacts 214 and 216. If contact 214 of switch 202 is deflected to touch contact 216, switch 202 is activated, i.e. electrical current is allowed between wires 208 and 210 through contacts 214 and 216. Wires 208 and 210 are in electrical connection with the electronic controls of control module 12 of apparatus 10. The electronic controls of control module 12 include suitable provisions for interrupting operation of pumping mechanism 62 when switch 202 is in an activated condition and may include various other provisions, such as an audible alarm, a visual indication in a liquid crystal display, an override for switch 202, and the like.

The operation and subtle features of occlusion detector 200 can now be set forth and appreciated. When reservoir module 14 is secured to control module 12, tube 22 of reservoir module 14 overlies switch 202 of control module 12 such that washer 212 abuts with and partially compresses tube 22. If the fluid within tube 22 is not under pressure, tube 22 is in a relaxed and unexpanded condition such that tube 22 does not deflect contact 14 to touch contact 16 and with switch 202 not being activated. On the other hand, if the fluid within tube 22 is under sufficient pressure, tube 22 is in an expanded condition and deflects contact 14 to touch contact 16 activating switch 202. It can then be appreciated that the amount of fluid pressure required in tube 22 to deflect contact 14 to touch contact 16 providing electrical connection depends on several factors including the spacing of switch 202 from pressure plate 20, the longitudinal thickness of washer 212, the spacing of contact 214 from the longitudinal end of washer 212, the spacing of contacts 214 and 216, the force required to deflect contact 214 into contact 216 and the like.

Prior to the present invention, occlusion was detected by a variety of methods. For example, prior infusion pumps included bladders or chamber members located within the pump which moved a piston or similar member which in turn actuated a switch. It can then be appreciated that the bladder or chamber member required special manufacture of the tube which delivers the medicant to the patient and also required the infusion pump to include a further multiplicity of parts which further increased the cost, complexity and size of the infusion pump. Other prior infusion pumps included plungers having enlarged head portions which bore against the tube which delivered the medicant to the patient. It can then be appreciated that this approach also required the infusion pump to include a further multiplicity of parts which also increased its cost, complexity and size. Other prior attempts included the provision of pressure diaphragms which were inserted in the tube at a location intermediate the infusion pump and the patient. Although this type of external approach allowed the infusion pump to be less complicated, such external, pressure diaphragms were relatively expensive to manufacture and rquired additional steps in the manufacture of the infusion pump at least because it was necessary to insert the pressure diaphragm into the medicant delivery tube. Other approaches have been utilized in prior infusion pumps which are similarly deficient and disadvantageous.

The present apparatus overcomes these deficiencies of the prior art by providing an occlusion detector 200 having switch 202 as part of control module 12 which directly acts upon tube 22 of reservoir module 14. Specifically, the proper interrelation between control module 12 and reservoir module 14 is insured by standoffs 40—43, the hinged connection including hooked portions 34 and hinge pins 128, and locking mechanism 146. Further, the proper interrelation of tube 22 beneath switch 202 is insured by standoff 40 and removed portion 48. Thus, automatic and foolproof placement and maintenance of tube 22 is created in a location allowing direct and accurate activation of switch 202.

Furthermore, no extra steps are required to adjust, calibrate, align, assemble or activate the occlusion detector 200, since occlusion detector 200 is fully functional and operable once reservoir module 14 is secured to control module 12. Thus, it can be appreciated that the modular construction of apparatus 10 including occlusion detector 200 is more user friendly than other prior drug delivery systems.

Additionally, tube 22 is of a uniform construction in its outer and inner diameters and its material throughout its length from drug storage container 18 of reservoir module 14 to adjacent the patient and does not require any bladder, chamber member or like specially manufactured portion as was required by prior infusion pumps. Furthermore, detector 200 of apparatus 10 does not require pistons, plungers or the like as were required by prior infusion pumps. Furthermore, apparatus 10 having an included occlusion detector 200 is not of any increased physical size and is of a simple design which is easy to manufacture. Thus, detector 200 is of a relatively low cost to manufacture as well as assemble.

Further, it should be realized that tube 22 with which switch 202 of detector 200 engages is a component of and is replaced with each reservoir module 14. Thus, tube 22 which is most subject to deformation and wear which may affect the accuracy of occlusion detector 200 is replaced with each medicant refill, greatly reducing the risk of equipment failure.

Many extensions and variations of the apparatus will be obvious to one having ordinary skill in the art. For example, it can be appreciated that reservoir module 14 can be formed of casing 16 having different heights to accommodate various sizes of bags 18. Likewise, it can be appreciated that pressure plate 20 can be adapted to support an external bag 18 which is not located in a generally closed casing 16.

**Claims**

1. Apparatus for delivering a drug to a patient, comprising a reservoir module (14) for holding a container (18) for storing the drug and means (22) for providing communication of the drug between the container and the patient, a control module (12) for controlling delivery of the drug, which control module is provided separably from said reservoir module and which comprises means (62) for forcing the drug from the container to the patient at desired rates, and means for removably securing the two modules together, characterised in that the reservoir module (14) further comprises a pressure plate (20) for supporting the communication providing means (22), and in that the drug forcing means (62) of the control module (12) operates by interaction with the communication providing means (22) located on the pressure plate (20) when the two modules are connected together.

2. Apparatus as claimed in claim 1 wherein the means for removably securing the reservoir module to the control module comprises means for removably attaching the reservoir module (14) to the control module (12) along one end, which removably attaching means has a first position wherein the reservoir module and the control module are separable from each other and a second position wherein the reservoir module and the control module are interconnected and nonseparable from each other, means being provided for holding the reservoir module and the control module in their second position.

3. Apparatus as claimed in claim 2 wherein the holding means comprises a locking mechanism (146).

4. Apparatus as claimed in claim 2 or claim 3 wherein the removably attaching means comprises hinge means comprising hinge pins (128) located on one of the reservoir module (14) and the control module (12) and hooked portions (34) complementary to and for receipt on the hinge pins located on the other of the reservoir module and the control module.

5. Apparatus as claimed in claim 1, or in claim 2 or claim 3 as dependent on claim 1, wherein the reservoir module (14) further comprises means for insuring proper spacing maintenance between the pressure plate (20) and the control module (12) for the efficient and advantageous interaction of the drug forcing means (62) of the control module (12) with the communication providing means (22).

6. Apparatus as claimed in claim 5 wherein the spacing maintenance insuring means comprises at least a first standoff (41) formed on the pressure plate (20) for abutting with the control module (12) when the reservoir module (14) and the control module are in their second position.

7. Apparatus as claimed in claim 6 wherein the drug forcing means (62) comprises a camshaft (74) having a first cam portion (76) and second and third cam portions (78 and 80) located on opposite sides of the first cam portion, an expulsor (86) reciprocably mounted in the control module (12) for engagement with the first cam portion and for interaction with the communication providing means (22), first and second valves (100, 102) reciprocably mounted in the control module for engagement with the second and third cam portions and for interaction with the communication providing means, and means for rotating the camshaft for reciprocating the expulsor and valves in the control module.

8. Apparatus as claimed in claim 7 wherein the drug forcing means (62) further comprises a chassis (64) which chassis includes a first aperture (88) for reciprocably receiving the expulsor (86) and second and third apertures (104, 106) located on opposite sides of the first aperture for reciprocably receiving the first and second valves (100, 102) and wherein the spacing maintenance insuring means further comprises a second standoff (42) with the first standoff (41) abutting with the chassis of the control module (12) between the first and second apertures and the second standoff abutting with the chassis of the control module between the first and third apertures.

9. Apparatus as claimed in claim 8 wherein the control module (12) further includes a casing (50), which casing has a generally closed top (52), a generally closed first sidewall (56), a generally closed second sidewall (57), a generally closed third sidewall (58), a generally closed fourth sidewall (59) and a generally open bottom (54), and the chassis (64) of the drug forcing means (62) has a size and shape complementary to and for receipt in the generally open bottom of the casing of the control module, and wherein the apparatus further comprises means (142) for sealing between the sidewalls of the casing and the chassis and means (90) for sealing between the apertures of the chassis and the expulsor (86) and valves (100, 102) whereby to form a substantially water tight compartment defined by the sidewalls and the top of the casing and the chassis of the control module.

10. Apparatus as claimed in any preceding claim wherein the communication providing means (22) comprises a tube (22) extending from the container (18) to the patient.

11. Apparatus as claimed in claim 10 wherein the tube (22) has a uniform construction in its outer and inner diameters and material throughout its length from the container to adjacent the patient.

12. Apparatus as claimed in claim 10 or claim 11 as dependent on claim 7, 8 or 9 wherein the standoffs (40—43) include first and second portions which are spaced apart a distance equal to and for receipt of the tube (22) therebetween to insure the tube is positioned on the pressure plate (20) to insure complete contact of the expulsor (86) and valves (100, 102) of the control module (12) with the tube at all times when the reservoir module (14) and the control module are located in their second position.

13. Apparatus as claimed in any preceding claim and further comprising an occlusion detector (200), which occlusion detector comprises a switch (202) mounted to the control module (12) for directly engaging and abutting with the communication providing means (22) of the reservoir module (14) intermediate the drug forcing means (62) and the patient, the switch being electrically connected to the drug forcing means for interrupting the forcing of the drug through the communication providing means when the pressure of the drug in the communication providing means is above a desired level.

14. Apparatus as claimed in claim 9 wherein the means for sealing between the apertures (88, 104, 106) of the chassis (64) and the expulsor (86) and the valves (100, 102) comprises a closed cell foam gasket (90) having apertures (108) corresponding to the first, second and third apertures of the chassis for the reciprocal receipt of the expulsor and the first and second valves, with the expulsor and the first and second valves having head portions (92, 110) for abutting with the gasket located on the chassis.

15. Apparatus as claimed in claim 14 wherein the gasket (90) has sufficient resiliency to bias the expulsor (86) and the first and second valves (100, 102) against the cam portions (76, 78, 80).

16. Apparatus as claimed in claim 3 wherein the locking mechanism comprises a latch button (150) having a longitudinal axis and a portion (178) eccentric to the longitudinal axis, means (148) for rotatably and slideably mounting the latch button about the longitudinal axis in the control module (12), means (176) for limiting the slideable movement of the latch button in the control module in a first direction, a locking bolt (152), which locking bolt is rotatably received on the eccentric portion of the latch button, means for preventing slideable movement of the locking bolt with respect to the latch button, means (190) for preventing the locking bolt from rotating wiht the latch button and for allowing slideable movement of the locking bolt with the latch button, means (156) for biasing the latch button in a second direction opposite to the first direction, and means (36) connected to the reservoir module (14) for engaging with the locking bolt, with the latch button having a first position wherein the locking bolt does not engage with the engaging means of the second module, a second, longitudinal position located longitudinally in the first direction from the first position, and a third, rotated position located longitudinally in the first direction from the first position and in a rotated position from the first and second positions allowing the locking bolt to engage with and place a force on the engaging means of the reservoir module.

17. Apparatus as claimed in claim 16 and further comprising means for releasably holding the latch button (15) in the third, rotated position.

18. Apparatus as claimed in claim 17 wherein the slideable movement preventing means comprises a shoulder formed on the latch button (150) adjacent the eccentric portion (178) with which the locking bolt (152) abuts, and a pin (154) extending through the latch button generally perpendicular to the longitudinal axis for abutting with the opposite side of the locking bolt than the shoulder.

19. Apparatus as claimed in claim 18 wherein the releasably holding means comprises a detent (172) formed in the control module (12) for receiving the pin (154) when the latch button (150) is in its third, rotated position.

20. Apparatus as claimed in claim 19 and further comprising a camming portion (166) formed in the control module (12) upon which the pin (154) rides between the first and third positions of the latch button (150).

21. Apparatus as claimed in claim 20, further comprising complementary tapered surfaces (189) formed on the locking bolt (152) and the engaging means (36) for enhancing the force placement on the engaging means of the reservoir module (14) by the locking bolt of the control module (12).

**Patentansprüche**

1. Gerät zum Zuführen eines Arzneimittels an einen Patienten, umfassend ein Speichermodul (14), das einen Container (18) trägt, der das Arzneimittel speichert und Mittel (22), die Verbindungswege für die Arzneimittel zwischen dem Container und dem Patienten vorsehen, ein Kontrollmodul (12), das die Zuführung des Arzneimittels überwacht, wobei das Kontrollmodul getrennt vom Speichermodul vorgesehen ist, und Mittel (62) umfaßt, die das Arzneimittel in gewünschten Mengen vom Container zum Patienten befördern, und Mittel zum sicheren Auswechseln der beiden Module zusammen, gekennzeichnet dadurch, daß das Speichermodul (14) außerdem eine Druckplatte (20) zum Abstützen der vorgesehenen Verbindungsmittel (22) umfaßt, und daß die Arznei Fördermittel (62) des Kontrollmoduls (12) durch das Zusammenwirken mit den vorgesehenen Verbindungsmitteln (22), arbeiten, die auf der Druckplatte (20) angeordnet sind, wenn die beiden Module miteinander verbunden sind.

2. Gerät nach Anspruch 1, wobei die Mittel zum lösbaren Befestigen des Speichermoduls am Kontrollmodul Mittel umfassen, zum lösbaren Befestigen des Speichermoduls (14) am Kontrollmodul (12) entlang eines Endes, wobei die auswechselbaren Befestigungsmittel eine erste Stellung haben, worin das Speichermodul und das Kontrollmodul getrennt voneinander sind und eine zweite Stellung, worin das Speichermodul und das Kontrollmodul miteinander verbunden sind und untrennbar voneinander sind, und Mittel, die vorgesehen sind das Speichermodul und das Kontrollmodul in ihrer zweiten Stellung zu halten.

3. Gerät nach Anspruch 2, wobei die Haltemittel einen Sperrmechanismus (146) umfassen.

4. Gerät nach Anspruch 2 oder 3, wobei die auswechselbaren Befestigungsmittel Scharnierelemente umfassen, die Scharnierstifte (128) enthalten, die auf dem Speichermodul (14) oder dem Kontrollmodul (12) angeordnet sind, und einen hakenförmigen Bereich (34) komplementär dazu und zur Aufnahme der Scharnierstifte, die auf dem anderen Speichermodul Kontrollmodul angeordnet sind.

5. Gerät nach Anspruch 1 oder Anspruch 2 oder Anspruch 3 als abhängige von Anspruch 1, wobei das Speichermodul (14) weiter Mittel umfaßt, um eine geeignete Abstandaufrechterhaltung zwischen der Druckplatte (20) und dem Kontrollmodul (12) für die effiziente und vorteilhafte Wechselwirkung der Arzneifördermittel (62) des Kontrollmoduls (12) mit den vorgesehenen Verbindungsmitteln (22) sicherzustellen.

6. Gerät nach Anspruch 5, wobei die Abstandaufrechterhaltungssicherungsmittel zumindest einen Ständer (41) umfassen, der auf der Druckplatte (20) ausgebildet ist zum Angrenzen an das Kontrollmodul (12) wenn das Speichermodul (14) und das Kontrollmodul in ihrer zweiten Position sind.

7. Gerät nach Anspruch 6, wobei die Arzneifördermittel (62) eine Nockenwelle (74) umfassen, die einen ersten Nockenbereich (76) und einen zweiten und dritten Nockenbereich (78 und 80) aufweist, die an gegenüberliegenden Seiten des ersten Nockenbereichs angeordnet sind, einen Auswerfer (86), der wechselseitig in dem Kontrollmodul (12) befestigt ist, zur Verbindung mit dem ersten Nockenbereich und zum Wechselwirken mit den vorgesehenen Verbindungsmitteln (22), ein erstes und zweites Ventil (100, 102), die reziprok in dem Kontrollmodul angeordnet sind für die Verbindung mit dem zweiten und dritten Nockenbereich und für das Zusammenwirken mit den Verbindungsmitteln, und Mittel zum Rotieren der Nockenwelle zum Hin- und Herbewegen des Auswerfers und der Ventile im Kontrollbereich.

8. Gerät nach Anspruch 7, wobei die Arzneifördermittel (62) weiter ein Chassis (64) umfassen, welches eine erste Öffnung (88) zum hin- und herbewegenden Aufnehmen des Auswerfers (86) beinhaltet und zweite und dritte Öffnungen (104, 106), die auf der gegenüberliegenden Seite zur ersten Öffnung angeordnet sind zum hin- und herbewegenden Aufnehmen des ersten und zweiten Ventils (100, 102) und worin die Abstandaufrechterhaltungssicherungsmittel weiter einen zweiten Ständer (42) umfaßt, wobei der erste Ständer (41) am Chassis des Kontrollmoduls (12) anliegt zwischen der ersten und zweiten Öffnung und der zweite Ständer am Chassis des Kontrollmoduls zwischen der ersten und dritten Öffnung liegt.

9. Gerät nach Anspruch 8, wobei das Kontrollmodul (12) weiter ein Gehäuse (50) beinhaltet, wobei das Gehäuse einen im allgemeinen geschlossenen Deckel (52) aufweist, eine im allgemeinen geschlossene erste Seitenwand (57), eine im allgemeinen geschlossene zweite Seitenwand (58), eine im allgemeinen geschlossene dritte Seitenwand (59) und einen im allgemeinen geschlossenen Boden (54), und das Chassis (64) des Arzneifördermittels (62) eine Größe und Form komplementär dazu hat zur Aufnahme im im allgemein offenen Boden des Gehäuses des Kontrollmoduls, und wobei das Gerät weitere Mittel (142) zum Abdichten zwischen der Seitenwand des Gehäuses und dem Chassis umfaßt und Mittel (90) zum Abdichten zwischen der Öffnung des Chassis und dem Auswerfer (86) und den Ventilen (100, 102), wodurch ein im wesentlichen wasserdichtes Schott gebildet wird, das durch die Seitenwände und den Deckel des Gehäuses und dem Chassis des Kontrollmoduls definiert ist.

10. Gerät nach den Ansprüchen 1 bis 9, wobei die vorgesehenen Verbindungsmittel (22) einen Schlauch (22) umfassen, der sich vom Container (18) bis zum Patienten erstreckt.

11. Gerät nach Anspruch 10, der abhängig ist von den Ansprüchen 7, 8 oder 9, wobei die Ständer (40 bis 43) erste und zweite Bereiche beinhalten, die in einem Abstand getrennt angeordnet sind zur Aufnahme des Schlauches (22) dazwischen, um sicherzustellen, daß der Schlauch auf der Druckplatte (20) positioniert ist, um völligen Kontakt des Auswerfers (86) und der Ventile (100, 102) des Kontrollmoduls (12) mit dem Schlauch zu jeder Zeit sicherzustellen, wenn das Speichermodul (14) und das Kontrollmodul (12) in der zweiten Position angeordnet sind.

12. Gerät nach den Ansprüchen 1 bis 11, und weiter einen Einschlußdetektor (200) umfassend, wobei der Einschlußdetektor einen Schalter (202) beinhaltet, der am Kontrollmodul (12) befestigt ist zum direkten Verbinden und Anliegen an die vorgesehenen Verbindungsmittel (22) des Speichermoduls (14) zwischen den Arzneifördermitteln (62) und dem Patienten, wobei der Schalter elektrisch verbunden ist mit den Arzneifördermitteln zum Unterbrechen des Förderns des Arzneimittels durch die Verbindungsmittel, wenn der Druck des Arzneimittels in den Verbindungsmitteln über einem gewünschten Niveau liegt.

13. Gerät nach Anspruch 9, wobei die Abdichtungsmittel zwischen der Öffnung (88, 104, 106) des Chassis (64) und dem Auswerfer (86) und den Ventilen (100, 102) eine geschlossene Zellenschaumdichtung (90) umfassen, die Öffnungen (108) aufweisen, die mit der ersten, zweiten und dritten Öffnung des Chassis übereinstimmen zur reziproken Aufnahme des Auswerfers und des ersten und zweiten Ventils, wobei der Auswerfer und das erste und zweite Ventil die einen Kopfbereich (92, 110) aufweisen, der an der Dichtung, die auf dem Chassis angeordnet ist anliegt.

14. Gerät nach Anspruch 13, wobei die Dichtung (90) ausreichend Spannkraft aufweist, um den Auswerfer (86) und das erste und zweiten Ventil (100, 102) gegen den Nockenbereich (76, 78, 80) zu drücken.

15. Gerät nach Anspruch 10, wobei der Schlauch (22) eine einheitliche Gestaltung in seinem äußeren und inneren Durchmesser und dem Material über seine Länge vom Container bis zum Patienten aufweist.

16. Gerät nach Anspruch 3, worin der Sperrmechanismus einen Verschließdrücker (150) umfaßt, der eine longitudinale Achse und einen Bereich (178) exzentrisch zur longitudinalen Achse aufweist, Mittel (148) zum drehbaren und schiebbaren Befestigen des Verschließdrückers über die longitudinale Achse im Kontrollmodul (12), Mittel (176) zum Begrenzen der schiebbaren Bewegung des Verschließdrückers im Kontrollmodul in einer ersten Richtung, einen Sperrbolzen (152), der drehbar in dem exzentrischen Bereich des Verschließdrückers aufgenommen ist, Mittel zum Vermeiden der schiebbaren Bewegung des Sperrbolzens in Bezug auf den Verschließdrücker, Mittel (190), die vermeiden, daß sich der Sperrbolzen mit dem Verschließdrücker dreht und schiebende Bewegungen des Sperrbolzens mit dem Verschließdrücker ermöglichen, Mittel (156) zum Vorspannen des Verschließdrückers in eine zweite Richtung entgegengesetzt der ersten Richtung, und Mittel (36), die mit dem Speichermodul (14) verbunden sind zum Angreifen am Sperrbolzen, wobei der Verschließdrücker eine erste Stellung hat, in der der Sperrbolzen nicht an den Angreifmitteln des zweiten Moduls angreift, eine zweite, longitudinale Stellung, die longitudinal an der ersten Richtung von der ersten Stellung angeordnet ist, und eine dritte, gedrehte Stellung, die longitudinal in der ersten Richtung von der ersten Stellung und in einer gedrehten Stellung von der ersten und zweiten Stellung angeordnet ist, die es dem Sperrbolzen ermöglicht, den Angreifmitteln des Speichermoduls daran anzugreifen und eine Kraft darauf auszuüben.

17. Gerät nach Anspruch 16 und weiter Mittel umfassend zum entlasteten Halten des Verschließdrückers (15) in der dritten, gedrehten Stellung.

18. Gerät nach Anspruch 17, wobei die Gleitbewegungsvermeidungsmittel eine Schulter umfassen, die auf dem Verschließdrücker (150) neben dem exzentrischen Bereich (178) an der der Sperrbolzen (152) angrenzt ausgebildet ist, und einen Stift (154), der sich durch den Verschließdrücker im wesentlichen senkrecht zur longitudinalen Achse erstreckt, um an der der Schulter gegenüberliegenden Seite des Sperrbolzens als der Schulter anzuliegen.

19. Gerät nach Anspruch 18, wobei die lösbaren Haltemittel einen Anschlagbolzen (172) umfassen, der im Kontrolmodul (12) ausgebildet ist zur Aufnahme des Stiftes (154), wenn der Verschließdrücker (150) in seiner dritten, gedrehten Stellung ist.

20. Gerät nach Anspruch 19 und weiter einen nockenartigen Bereich (166) umfassend, der im Kontrolmodul (12) ausgebildet ist und worauf der Stift zwischen der ersten und dritten Stellung des Verschließdrückers (150) gleitet.

21. Gerät nach Anspruch 20, weiter umfassend komplementäre konische Oberflächen (189), die auf dem Sperrbolzen (152) und den Angreifmitteln (36) ausgebildet sind zum Steigern der Kraftausübung auf die Angreifmittel des Speichermoduls (14) durch die Sperrbolzen des Kontrollmoduls (12).

## Revendications

1. Appareil ou système pour l'administration ou la délivrance d'un médicament à un patient, cet appareil comprenant un module (14) réservoir pour loger un récipient (18) destiné à stocker le médicament, et un moyen (22) pour assurer la communication du médicament entre le récipient et le patient, un module (12) de commande pour commander la délivrance du médicament, ce module de commande étant fourni séparément dudit module réservoir et comprenant un moyen (62) pour refouler le médicament du récipient vers le patient à des débits voulus, et un moyen pour fixer amoviblement ensemble les deux modules, appareil caractérisé en ce que le module (12) réservoir comprend en outre une plaque (20) d'appui destiné à supporter le moyen (22) assurant la communication, et en ce que le moyen (62), destiné à refouler le médicament et faisant partie du module (12) de commande, fonctionne par interaction avec le moyen (22) assurant la communication et situé sur la plaque (20) d'appui, lorsque les deux modules sont connectés ensemble.

2. Appareil tel que revendiqué à la revendication 1, dans lequel le moyen pour fixer amoviblement le module réservoir au module de commande comprend un moyen pour attacher amoviblement le module (14) réservoir au module (12) de commande le long d'une extrémité, ce moyen de fixation amovible comportant une première position dans laquelle le module réservoir et le module de commande peuvent être séparés l'un de l'autre et une seconde position dans laquelle le module réservoir et le module de commande sont interconnectés et ne peuvent être séparés l'un de l'autre, un moyen étant prévu pour maintenir le module réservoir et le module de commande dans leur seconde position.

3. Appareil tel que revendiqué à la revendication 2, dans lequel le moyen de maintien comprend un mécanisme (146) de verrouillage.

4. Appareil tel que revendiqué à la revendication 2 ou à la revendication 3, dans lequel le moyen de fixation amovible comprend un moyen d'articulation comprenant des axes (128) d'articulation situés sur l'un des modules, le module (14) réservoir ou le module (12) de commande, et des parties (34), racourbées en crochets, complémentaires des axes d'articulation et destinées à recevoir ces axes situés sur l'autre module, le module réservoir ou le module de commande.

5. Appareil tel que revendiqué à la revendication 1 ou à la revendication 2 ou la revendication 3 dépendant de la revendication 1, dans lequel le module (14) réservoir comprend en outre un moyen pour garantir le maintien d'un espacement approprié entre la plaque (20) d'appui et le module (12) de commande pour l'interaction efficace et avantageuse du moyen (62) de refoulement du médicament, faisant partie du module (12) de commande, avec le moyen (22) assurant la communication.

6. Appareil tel que revendiqué à la revendication 5, dans lequel le moyen garantissant le maintien d'un espacement comprend au moins un premier organe (41) de séparation formé sur la plaque (20) d'appui pour venir en aboutement avec le module (12) de commande lorsque le module (14) réservoir et le module de commande sont en leur seconde position.

7. Appareil tel que revendiqué à la revendication 6, dans lequel le moyen (12) de refoulement du médicament comprend un arbre (74) à cames comprenant une première partie (76) de came et des seconde et troisième parties (78 et 80) de came situées sur des côtés opposés de la première partie de came, un organe (86) d'expulsion monté dans le module (12) de commande pour y exécuter un mouvement alternatif et venir au contact de la première partie de came et pour interagir avec le moyen (22) assurant une communication, des première et seconde soupapes (100, 102) montées dans le module de commande de façon à exécuter un mouvement alternatif et à venir au contact des seconde et troisième parties de came et pour interagir avec le moyen assurant la communication, et un moyen pour faire tourner l'arbre de came afin de conférer un mouvement alternatif à l'organe d'expulsion et aux soupapes dans le module de commande.

8. Appareil tel que revendiqué à la revendication 7, dans lequel le moyen (62) pour refouler le médicament comprend en outre un châssis (64), ledit châssis comprenant une première ouverture (88) destinée à recevoir alternativement l'organe (86) d'expulsion, et des seconde et troisième ouvertures (104, 106), situées sur des côtés opposés de la première ouverture pour recevoir alternativement les première et seconde soupapes (100, 102), et le moyen destiné à garantir le maintien de l'espacement comprend en outre un second organe (42) de séparation, le premier organe (41) de séparation venant buter sur le châssis du module (12) de commande entre les première et seconde ouvertures et le second organe de séparation venant buter sur le châssis du module de commande entre les première et troisième ouvertures.

9. Appareil tel que revendiqué à la revendication 8, dans lequel le module (12) de commande comprend en outre un boîtier (50), ce boîtier ayant un sommet au-dessus (52) fermé de manière générale, une première paroi latérale (56) fermée de manière générale, une seconde paroi latérale (57) fermée de manière générale, une troisième paroi latérale (58) fermée de manière générale, une quatrième paroi latérale (59) fermée de manière générale et un fond (54) ouvert de manière générale, et le châssis du moyen (62) de refoulement du médicament présente une taille et une forme complémentaires de celles du fond généralement ouvert du boîtier du module de commande, de façon à pouvoir être reçu et logé dans ce fond de boîtier; et l'appareil comprend en outre un moyen (142) pour assurer l'étanchéité entre les parois latérales du boîtier et le châssis, et un moyen (90) pour assurer l'étanchéité entre les ouvertures du châssis et l'organe (86) d'expulsion et les soupapes (100, 102), de manière à former un compartiment essentiellement étanche à l'eau et délimité par les parois latérales et le sommet du boîtier et le châssis du module de commande.

10. Appareil tel que revendiqué dans l'une quelconque des revendications précédentes, dans lequel le moyen (22) assurant la communication comprend un tube (22) s'étendant du récipient (18) jusqu'au patient.

11. Appareil tel que revendiqué à la revendication 10, dans lequel le tube (22) a une structure uniforme pour ses diamètres extérieur et intérieur et pour sa matière sur toute sa longueur du récipient jusqu'au voisinage du patient.

12. Appareil tel que revendiqué à la revendication 10 ou à la revendication 11 lorsqu'elles dépendent de la revendication 7, 8 ou 9, dans lequel les organes (40 à 43) de séparation comprennent des première et seconde parties qui sont séparées d'une distance égale au diamètre du tube (22) pour loger ce tube (22) entre elles afin de garantir que le tube soit placé sur la plaque (20) d'appui pour garantir un contact complet de l'organe (86) d'expulsion et des soupapes (100, 102) du module (12) de commande avec le tube à tout moment lorsque le module (14) réservoir et le module de commande sont situés en leur seconde position.

13. Appareil tel que revendiqué dans l'une quelconque des revendications précédentes et comportant en outre un détecteur (200) d'occlusion, ce détecteur d'occlusion comprenant un commutateur (202) monté sur le module (12) de commande pour venir directement en contact et en butée sur le moyen (22) assurant la communication du module (14) réservoir entre le moyen (62) pour refouler le médicament et le patient, le commutateur étant électriquement connecté au moyen de refoulement du médicament pour interrompre le refoulement du médicament dans le moyen assurant la communication lorsque la pression du médicament, dans le moyen assurant la communication, est supérieure à un niveau voulu.

14. Appareil tel que revendiqué à la revendication 9, dans lequel le moyen pour assurer l'étanchéité entre les ouvertures (88, 104, 106) du châssis (64) et l'organe (86) d'expulsion et les soupapes (100, 102) comprend un joint de garniture (90) en mousse à cellules ou pores fermés, ayant des ouvertures (108) correspondant aux première, seconde et troisième ouvertures du châssis pour la réception de logement alternatif de l'organe d'expulsion et des première et seconde soupapes, l'organe d'expulsion et les première et seconde soupapes comportant des parties (92, 110) de tête destinées à venir affleurer le joint de garniture situé sur la châssis.

15. Appareil tel que revendiqué à la revendication 14, dans lequel le joint de garniture (90) a suffisamment d'élasticité pour pousser l'organe (86) d'expulsion et les première et seconde soupapes (100, 102) contre les parties (76, 78, 80) de cames.

16. Appareil tel que revendiqué à la revendication 3, dans lequel le mécanisme de verrouillage comprend un bouton (150) de verrouillage ayant un axe longitudinal et une partie (178) excentrique par rapport à l'axe longitudinal, un moyen (148) pour monter, de façon à permettre une rotation et un glissement, le bouton de verrouillage autour de l'axe longitudinal dans le module (12) de commande, un moyen (176) pour limiter le mouvement de coulissement du bouton de verrouillage, dans le module de commande, dans une première direction, un boulon (152) de verrouillage, qui est logé de façon à pouvoir tourner sur la partie excentrique du bouton de verrouillage, un moyen pour empêcher le mouvement de glissement ou coulissement du boulon de verrouillage par rapport au bouton de verrouillage, un moyen (190) pour empêcher le boulon de verrouillage de tourner avec le bouton de verrouillage et permettre un mouvement de coulissement du boulon de verrouillage avec le bouton de verrouillage, un moyen (156) pour pousser le bouton de verrouillage dans une seconde direction opposée à la première direction, et un moyen (36), relié au module (14) réservoir pour venir au contact du boulon de verrouillage, le bouton de verrouillage ayant une première position dans laquelle le boulon de verrouillage ne vient pas au contact du moyen de venue en contact du second module, une seconde position longitudinale située longitudinalement dans la première direction par rapport à la première position, et une troisième position avec rotation située longitudinalement dans la première direction par rapport à la première position et dans une position de rotation par rapport aux première et seconde positions, ce qui permet au boulon de verrouillage de venir au contact de l'organe de venue au contact du module réservoir et d'exercer une force sur ce dernier moyen.

17. Appareil tel que revendiqué à la revendication 16, et comprenant en outre un moyen pour maintenir amoviblement le bouton (15) de verrouillage dans la troisième position obtenue avec rotation.

18. Appareil tel que revendiqué à la revendication 17, dans laquelle le moyen empêchant un mouvement de glissement comprend un épaulement formé sur le bouton (150) de verrouillage près de la partie excentrique (178) avec laquelle le boulon (152) de verrouillage vient en contact, et une tige (154) qui s'étend à travers le bouton de verrouillage, de manière généralement perpendiculaire à l'axe longitudinal, pour venir buter sur le côté du boulon de verrouillage opposé à l'épaulement.

19. Appareil tel que revendiqué à la revendication 18, dans lequel le moyen permettant d'assurer un effet de retenue permettant un dégagement comprend un organe (172) de butée, formé dans le module (12) de commande pour recevoir la tige (154) lorsque le bouton (150) de verrouillage se trouve dans sa troisième position obtenue par rotation.

20. Appareil tel que revendiqué à la revendication 19 et comprenant en outre une partie (166) formant came, formée dans le module (12) de commande et que la tige (154) chevauche entre les première et troisième positions du bouton (150) de verrouillage.

21. Appareil tel que revendiqué à la revendication 20, comprenant en outre des surfaces (189) inclinées de manière complémentaire, formées sur le boulon (152) de verrouillage et sur le moyen (36) de venue en contact pour augmenter l'effort exercé par le boulon de verrouillage du module (12) de commande sur le moyen de venue en contact du module (14) réservoir.

*Fig 1*

*Fig 3*

*Fig 4*

Fig 2

Fig 5

2

Fig 6

Fig 7

Fig 8